# EUROPEAN PATENT SPECIFICATION

(11) **EP 2 513 327 B1**
(45) Date of publication and mention of the grant of the patent: **29.06.2016**
(21) Application number: 10794956.2
(22) Date of filing: 14.12.2010
(51) Int. Cl.: C12P 35/04

(54) **PRODUCTION PROCESS FOR CEPHRADINE**
HERSTELLUNGSVERFAHREN FÜR CEPHRADIN
PROCÉDÉ DE FABRICATION DE LA CÉPHRADINE

(30) Priority: 14.12.2009 EP 09179015
(43) Date of publication of application: 24.10.2012
(73) Proprietor: DSM Sinochem Pharmaceuticals Netherlands B.V., 2613 AX Delft (NL)
(72) Inventor: MOODY, Harold, Monro, NL-6271 ED Gulpen (NL); DOOREN, VAN, Theodorus, Johannes, Godfried, Maria, NL-6042 BZ Roermond (NL)
(74) Representative: De Vroom, Erik
(86) International application number: PCT/EP2010/069575
(87) International publication number: WO 2011/073166

(56) References cited:
- WO-A1-97/04086
- WO-A1-03/055892
- WO-A1-2008/110527
- WO-A2-2005/003367
- US-A- 5 034 522

## Description

### Field of the invention

The present invention relates to an enzymatic process for the preparation of cephradine under anaerobic conditions.

### Background of the invention

In a chemical process for the production of cephradine, the amino-group in 7-aminodesacetoxy-cephalosporanic acid (7-ADCA) is acylated with a dihydrophenylglycine (DHPG) side chain while dichloromethane is used as solvent. For example, D(-)-α-2,5-dihydrophenylglycine-methyl-sodium-Dane salt and pivaloyl chloride in dichloromethane may be added to a solution of 7-ADCA and 1,8-diazabicyclo-(5,4,0)-undec-7-ene (DBU) in dichloromethane. The resulting reaction mixture comprises cephradine with protected groups. The use of protected intermediates, such as cephradine with protecting groups, is characteristic for a chemical process for the production of cephalosporins, for example cephradine. To remove the protecting groups, water and HCl are added to the reaction mixture. The reaction mixture and added acidic solution form a two phase system, which is subsequently separated into two separate phases, an organic phase comprising mainly dichloromethane and pivalic acid with some residual water and an aqueous phase comprising water, cephradine and HCl in solution, as well as residual dichloromethane. Typically, the pH of the acidic aqueous solution, obtained through said chemical process for the production of cephradine and subsequent separation of the reaction mixture, is below 3.2. Subsequently, the formed cephradine is recovered from the aqueous phase by a pH shift; a complexing agent may be added. For example DMF or quinoline can be added to produce a solid cephradine complex, which is subsequently converted to cephradine.

The great disadvantage of chemical synthesis of semi-synthetic antibiotics in general and cephradine in particular is, that these chemical processes are carried out using organic solvents such as dichloromethane and DMF. This makes these processes environmentally unfriendly since a lot of chemical/solvent waste is produced.

A major breakthrough in the synthesis of semi-synthetic antibiotics came with the introduction of enzymes that are capable of coupling the side chain (e.g. PG, HPG, DHPG, all in activated form as amide or ester) to the nuclei (e.g. 7-ADCA or 6-APA) in an aqueous environment thereby avoiding the consumption of organic solvents.

WO97/04086 discloses the enzymatic production of β-lactam antibiotics by reaction of a parent β-lactam with a side chain in activated form, including the preparation of cephradine by reaction of 7-ADCA with DHPG in activated form (DHPGa). This publication describes that besides the synthesis reaction, i.e. the reaction of the activated side chain with the parent β-lactam, also side chain acids are formed by hydrolysis of the activated side chain and the desired product. The side chain acid formed in case of the enzymatic production of cephradine is D-dihydrophenylglycine (DHPG). WO97/04086 discloses *inter alia* the enzymatic synthesis of cephradine in the presence of wild-type acylase of *E. coli* and shows that when penicillin acylase was immobilised on a specific carrier, an increased synthesis/hydrolysis (S/H) ratio was obtained. In the process of WO97/04086 conversions of 7-ADCA and DHPG-methyl ester into cephradine of up to 68% had been achieved. In WO2005/003367, the enzymatic process of WO97/04086 is further improved resulting in a conversion of 7-ADCA into cephradine of at least 70% while keeping the concentration D-dihydrophenylglycine (DHPG) in the reaction mixture below 2 wt.%.

The Pharmacopeia dictates that cephradine, when it is used as an active pharmaceutical ingredient (API), should not contain more than 5.0% cephalexin. The molecular structure of cephalexin very much resembles that of cephradine, the only difference being the side chain: DHPG in the case of cephradine and PG in the case of cephalexin.

Cephradine is not stable and, under the influence of oxygen, is converted into cephalexin. For this reason, cephradine, after its industrial production, is usually stored and handled under such conditions that oxidation is minimized. For instance, a suitable packaging is under vacuum in sealed bags. Usually, cephradine obtained after the production process should contain less than the 5.0% dictated by the Pharmacopeia in order to allow some formation of cephalexin during storage of the cephradine until its ultimate expiry date.

Another reason for the presence of cephalexin in cephradine is that DHPG, used in the synthesis, is already contaminated with some PG. For industrial grade DHPG, the amount of PG may be up to 2% and usually is around 1%. Also DHPG is not stable and should be stored and handled with caution to prevent the oxidation to PG. Chemical synthesis of cephradine results in a product having about 1.0-1.5% cephalexin.

The inventors have now found that under conditions of the enzymatic synthesis of cephradine from 7-ADCA and DHPG in activated form and also during the activation of DHPG, substantial more oxidation may take place which results in cephradine containing too high levels of cephalexin compared to the chemical synthesis route. Levels higher than those prescribed by the Pharmacopeia were obtained, for example between 5-10%. It is therefore an object of the present invention to provide a process for the enzymatic synthesis of cephradine from 7-ADCA and DHPG under such conditions that cephradine is obtained which, also after storage, still complies with the provisions of the Pharmacopeia.

### Detailed description of the invention

### List of abbreviations

- 6-APA:: 6-aminopenicillanic acid
- 7-ADCA:: 7-aminodesacetoxy-cephalosporanic acid
- DHPG:: D-dihydrophenylglycine
- DHPGa:: D-dihydrophenylglycine in activated form
- DHPGM:: D-dihydrophenylglycine methyl ester
- DMF:: N,N-dimethylformamide
- DCME:: Dichloromethane
- HPG:: D-hydroxyphenylglycine
- PG:: D-phenylglycine
- PGM:: D-phenylglycine methyl ester

The invention provides a process for preparing cephradine, said process comprising (a) converting D-dihydrophenylglycine (DHPG) into an activated form (DHPGa) which is an amide or an ester and (b) reacting 7-aminodesacetoxy-cephalosporanic acid (7-ADCA) with D-dihydrophenylglycine in activated form (DHPGa) in the presence of a penicillin acylase in an aqueous reaction mixture to form cephradine, characterized in that at least step (a) and step (b) of the process are carried out under anaerobic conditions. The advantage of the process of the invention is that cephradine is obtained with contains lower amounts of cephalexin compared to the same process which is not being carried out under anaerobic conditions.

Anaerobic conditions are defined herein as those conditions at which at least one, or several or preferably all steps in the process for the production of cephradine are carried out in the presence of oxygen levels which have been reduced at least by 50%, preferably by at least 75%, more preferably by at least 90%, most preferably by at least 95% compared to the oxygen level under not anaerobic conditions, most preferably in the absence of oxygen. There are various measures that can be used to obtain the anaerobic conditions as defined above:
a. The process may be carried out in the presence of oxygen scavengers, i.e. compounds which react with oxygen such that the effective concentration of the reactive oxygen is decreased. Suitable oxygen scavengers are ascorbic acid, metabisulphite, dithiothreitol, mercaptoethanol, dithiotrite and bisulphite. Most preferred is bisulphite.
b. One or more of the raw materials, reagents, solvents and/or water used in the process may be degassed. A suitable method for degassing is bringing the raw materials, reagents, solvents and/or water under vacuum conditions such that the oxygen is removed; alternatively, the raw materials, reagents, solvents and/or water may be flushed with an inert gas to replace the oxygen. Suitable inert gases are nitrogen, helium, argon and others. Most preferred is nitrogen.
c. The process may be carried out in an atmosphere comprising an inert gas. Suitable inert gases are nitrogen, helium, argon and others. Most preferred is nitrogen.

Preferably the process is carried out by applying any combination of the measures (a), (b) and (c) listed above, for instance (a) + (b) or (a) + (c) or (b) + (c). Most preferably the process is carried out by applying all measures (a) + (b) + (c).

In the process of the invention, the DHPGa in the activated form may be an amide or an ester, preferably DHPGa is an ester, such as the methyl ester or the ethyl ester. In a preferred embodiment of the process invention, DHPG is converted to an ester by contacting DHPG with an alcohol to form a mixture comprising the corresponding side chain ester. The alcohol used in the process of the invention may be selected from the group consisting of methanol and ethanol thereby forming the methyl ester and ethyl ester of the side chain respectively.

Step (b) of the process may be carried out as disclosed in WO2005/003367 provided that it is carried out under anaerobic conditions according to the process of the present invention. Thus, step (b) may be carried out by anaerobically reacting 7-ADCA with DHPGa which may result in a conversion of DHPGa into cephradine of at least 70%, preferably at least 80%, more preferably at least 90%, wherein conversion of DHPGa into CEF = (nCEF / nDHPGa)*100%, wherein nCEF = total quantity of cephradine formed (in mole); and nDHPGa = total quantity of DHPGa added to reaction mixture (in mole).

The DHPGa in the reaction mixture may be added in many ways. All the DHPGa may be added, initially, i.e. at the start of the reaction, for instance in a batch process. It is also possible to add at least part of the DHPGa to the reaction mixture during the course of the reaction.

The concentration DHPG in the reaction mixture may be maintained below 2 wt.% throughout the reaction, preferably below 1.5 wt.%, preferably below 1 wt.%, more preferably below 0.8 wt.%, e.g. by controlling the pH of the reaction mixture and/or the temperature. The pH and temperature applied may vary between wide ranges. The reaction to form the cephradine may for instance be carried out at a temperature of between -5 and 35°C, preferably between 0 and 30°C. More preferably, the reaction is carried out at a temperature of between 5°C and 25°C. The reaction to form the cephradine may for instance be carried out at a pH of between 6 and 9. Preferably, the reaction is carried out at a pH of between 6.3 and 8.5. The pH of the reaction mixture may be adjusted at the desired pH value in several ways, for instance chemically by adding an acid, for instance a mineral acid, in particular sulphuric acid, hydrochloric acid or nitric acid. The pH may also be adjusted at the desired pH value by adding a base, for instance sodium hydroxide or ammonia.

The concentration DHPG in the reaction mixture may also be maintained below 2 wt.% throughout the reaction, preferably below 1.5 wt.%, preferably below 1 wt.%, more preferably below 0.8 wt.% by working at diluted concentrations of the reactants 7-ADCA and DHPGa or by using an enzyme with improved properties, for instance an improved S/H-ratio, which may be a wild type or mutant enzyme. Maintaining the concentration of DHPG low according to the invention allows the conversion to be increased. This can then for instance be achieved by applying a residence time that is sufficiently long.

The process of the invention including step (a) and (b) may also be carried out as disclosed in WO 2008/110527 provided that it is carried out under anaerobic conditions according to the process of the present invention. In this process, the DHPGa which is anaerobically formed in step (a) according to the process of the present invention, is not isolated as a solid intermediate. The process may for instance comprise the following steps:
a) anaerobically converting DHPG with an alcohol to form a mixture comprising the corresponding side chain ester; the formation of the side chain ester in this step preferably results in a mixture with a conversion which is expressed by the "ratio" which is defined as:

   [DHPG-ester]

   [DHPG-ester] + [DHPG]

   and whereby the ratio is preferably ≥85%, more preferably ≥90%, more preferably ≥95%, more preferably ≥96%, more preferably ≥97%, more preferably ≥98%, most preferably ≥99%;
b) anaerobically forming the cephradine by mixing the mixture obtained in step a) with 7-ADCA and the enzyme to form cephradine, with the proviso that the side chain ester formed in step (a) is not isolated as a solid intermediate.

In the conversion step (step (a)) of the process of the invention DHPG is converted with an alcohol to form a mixture comprising the corresponding side chain ester under anaerobic conditions. The alcohol used in the process of the invention may be selected from the group consisting of methanol and ethanol thereby forming the methyl ester and ethyl ester of the side chain respectively. The most preferred alcohol is methanol. Step (a) may be carried out in several ways.

One embodiment of conversion step (a) comprises heating of a mixture comprising a free side chain selected from the group consisting of D-phenylglycine and DHPG, an alcohol selected on the basis of the desired ester to be formed, for example methanol to obtain the methyl ester or ethanol to obtain the ethyl ester, and a strong acid such as sulfuric acid, under reflux at a temperature between 20 and 120°C, more preferably between 40 and 100°C. When using methanol as the alcohol, the temperature is preferably between 60 and 80°C. When using ethanol as the alcohol, the temperature is preferably between 65 and 100°C. Suitable conditions may be found in the comparative examples 1 and 2 as disclosed in EP-A-0544205.

Another embodiment of conversion step (a) comprises an improvement of the previous embodiment and comprises addition of the alcohol as a liquid or a gas to the reaction mixture while distilling off the alcohol and the water from the reaction (e.g. as described in EP-A-0544205).

A highly preferred embodiment of conversion step (a) and which results in a mixture comprising DHPGMe comprises the following steps:
1. refluxing a reaction mixture comprising DHPG, methanol and a strong acid such as sulfuric acid for a certain time, for example between 0.5 and 5 hours, preferably between 1 hour and 3 hours, more preferably between the 1.5 and 2.5 hours at a temperature in the temperature between 20 and 120°C, more preferably between 20 and 100°C, more preferably between 40 and 100°C, most preferably between 60 and 80°C; followed by
2. concentrating the mixture at a temperature between 40 and 100°C, preferably between 60 and 90°C, more preferably between 70 and 80°C. The pressure during this step may initially be atmospheric and may during the concentrating step be reduced to preferably 50 mbar or less, more preferably to 40 mbar or less, more preferably to 30 mbar or less and most preferably to 20 mbar or less. The concentrating step is continued until more than 30% of the water present before the concentrating step is removed, preferably more than 40% of the water is removed, preferably more than 50% of the water is removed, preferably more than 70% of the water is removed, preferably more than 80% of the water is removed and most preferably more than 90% of the water is removed.
3. adding methanol, preferably an amount so as to obtain the initial volume of the reaction mixture before the concentrating step or an amount which is less than the initial volume of the reaction mixture, e.g. ≤90% or ≤80% or ≤70% or ≤60% or ≤50% or ≤40% or ≤30% or ≤20% of the initial volume of the reaction mixture. The amount of methanol added may also be more than the initial volume of the reaction mixture.
4. optionally repeating steps 1-3 one or more times, preferably at least once, preferably 2 times, more preferably 3 times, more preferably 4 times, more preferably 5 times, more preferably 6 times, more preferably 7 times, more preferably 8 times, more preferably 9 times, more preferably 10 times. It was found that by repeating these steps the "ratio" as defined hereinbefore of the formation of methyl ester increased significantly. For instance, after carrying out steps (a) - (c) only once, a "ratio" between 75-85% may be obtained, after repeating steps (a) - (c) once, a "ratio" between 85-95% may be obtained, and after repeating steps (a) - (c) 2 times, a "ratio" between 95-97% may be obtained, and after repeating steps (a) - (c) 3 times, a "ratio" between 97-98% may be obtained and after repeating steps (a) - (c) 4 times, a "ratio" between 98-99% may be obtained and after repeating steps (a) - (c) 5 times, a "ratio" between 99-99.5% may be obtained and after repeating steps (a) - (c) more than 5 times a "ratio of more than 99.5% may be obtained.

Another highly preferred embodiment of conversion step (a) and which results in a mixture comprising the ethyl ester of the side chain comprises the following steps:
1. refluxing a reaction mixture comprising DHPG, ethanol and a strong acid such as sulfuric acid for a certain time, for example between 0.5 and 5 hours, preferably between 1 hour and 3 hours, more preferably between the 1.5 and 2.5 hours at a temperature in the temperature between 20 and 120°C, more preferably between 20 and 100°C, more preferably between 40 and 100°C, most preferably between 65 and 100°C; followed by
2. concentrating the mixture at a temperature between 40 and 100°C, preferably between 60 and 90°C, more preferably between 70 and 80°C. The pressure during this step may initially be atmospheric and may during the concentrating step be reduced to preferably 50 mbar or less, more preferably to 40 mbar or less, more preferably to 30 mbar or less and most preferably to 20 mbar or less. The concentrating step is continued until more than 30% of the water present before the concentrating step is removed, preferably more than 40% of the water is removed, preferably more than 50% of the water is removed, preferably more than 70% of the water is removed, preferably more than 80% of the water is removed and most preferably more than 90% of the water is removed.
3. adding ethanol, preferably an amount so as to obtain the initial volume of the reaction mixture before the concentrating step or an amount which is less than the initial volume of the reaction mixture, e.g. ≤90% or ≤80% or ≤70% or ≤60% or ≤50% or ≤40% or ≤30% or ≤20% of the initial volume of the reaction mixture. The amount of ethanol added may also be more than the initial volume of the reaction mixture.
4. optionally repeating steps 1-3 one or more times, preferably at least once, preferably 2 times, more preferably 3 times, more preferably 4 times, more preferably 5 times, more preferably 6 times, more preferably 7 times, more preferably 8 times, more preferably 9 times, more preferably 10 times. It was found that by repeating these steps the "ratio" as defined hereinbefore of the formation of ethyl ester increased significantly. For instance, after carrying out steps (a) - (c) only once, a "ratio" between 75-85% may be obtained, after repeating steps (a) - (c) once, a "ratio" between 85-95% may be obtained, and after repeating steps (a) - (c) 2 times, a "ratio" between 95-97% may be obtained, and after repeating steps (a) - (c) 3 times, a "ratio" between 97-98% may be obtained and after repeating steps (a) - (c) 4 times, a "ratio" between 98-99% may be obtained and after repeating steps (a) - (c) 5 times, a "ratio" between 99-99.5% may be obtained and after repeating steps (a) - (c) more than 5 times a "ratio of more than 99.5% may be obtained.

In all embodiments of step (a), preferably the following molar ratio of alcohol versus DHPG is used: between 3 and 25, more preferably between 5 and 25 and most preferably between 6 and 10. Also, in all embodiments of step (a), preferably the following molar ratio of strong acid (in equivalents, e.g. one mole of hydrochloric acid is one equivalent and one mole of sulfuric acid is two equivalents) versus free side chain is used: between 0.9 and 10, more preferably between 1 and 5 and most preferably between 2 and 3. The skilled person will be able to optimize the reaction conditions depending on the side chain and the alcohol selected without undue experimentation.

Before forming cephradine in step (b), the mixture obtained in step (a) may be purified so as to obtain a mixture with a high "ratio" as defined hereinbefore. The ratio of the mixture which is to be used in step (b) of the process of the invention is preferably ≥85%, more preferably ≥90%, more preferably ≥95%, more preferably ≥96%, more preferably ≥97%, more preferably ≥98%, most preferably ≥99%.

One embodiment of the purification step involves the precipitation and removal of the DHPG from the DHPG-ester. This may be achieved by adjusting the pH of the mixture obtained in step (a) to a value between 2 and 6.5, preferably between 2.5 and 5, most preferred between 3 and 4 by adding a suitable base, such as NaOH, ammonia, KOH. In another embodiment, the reaction mixture obtained in step (a) may be added to a suitable amount of water or to an alcohol or to a mixture of water and alcohol, followed by adjusting the pH to a value between 2 and 6.5, preferably between 2.5 and 5, most preferred between 3 and 4 by adding a suitable base, such as NaOH, ammonia, KOH. After adjusting the pH to the desired value, the pH may be maintained at the desired value by adding the suitable base. Under these conditions, a precipitate comprising the free side chain may be formed. After a suitable time, the precipitate may be filtered off using known techniques. The filtrate comprises the side chain ester. In order to be used directly in step (b) of the process of the invention, the pH of the filtrate is brought to a pH between 1 and 6, preferably between 1 and 4, most preferably between 1.5 and 3, after which the alcohol is removed by evaporation using known techniques.

Another embodiment of the purification step involves the formation of a two or multi-phase system comprising an organic phase containing the side chain ester derivative and a minor amount of free side chain and an aqueous phase containing the free side chain and, optionally, salt. This may be achieved by adjusting the pH of the mixture obtained in step (a) at a value between 7.5 and 10, preferably between 8.5 and 9.5, most preferred between 8.8 and 9.2 by adding a suitable base, such as NaOH, ammonia, KOH. In another embodiment, the reaction mixture obtained in step (a) may be added to a suitable amount of water, an alcohol or to a mixture of water and alcohol, followed by adjusting the pH at a value between 7.5 and 10, preferably between 8.5 and 9.5, most preferred between 8.8 and 9.2 by adding a suitable base, such as NaOH, ammonia, KOH. After adjusting the pH to the desired value, the pH may be maintained at the desired value by adding the suitable base. Optionally, the water may be in the form of an aqueous salt (e.g. NaCl) solution. The free side chain may also form a precipitate. The various phases in the multi-phase system may be separated using known techniques. Optionally the organic phase may be washed with water or an aqueous salt solution. The water phase of the wash may be recycled to a suitable process stream in order to avoid loss of yield. This process stream may be the reaction mixture as obtained after step (a) or the after the pH adjustment as described.

A highly preferred embodiment of purification step combines the two previous embodiments, i.e. first adjusting the pH of the mixture obtained in step (a) between 2 and 6.5, preferably between 2.5 and 5, most preferred between 3 and 4 and filtering off the precipitate formed and subsequently adjusting the pH of the filtrate obtained at a pH between 7.5 and 10, preferably between 8.5 and 9.5, most preferred between 8.8 and 9.2 and separating the various phases in the multi-phase system obtained using known techniques.

It has been surprisingly found that in the previous two embodiments wherein the multi-phase system is formed at a pH between 7.5 and 10, preferably between 8.5 and 9.5, most preferred between 8.8 and 9.2, may yield an ester, preferably selected from the group consisting of DHPG-methylester and DHPG-ethylester, in the free base form, whereby the ester has the following properties:
- an e.e. (enantiomeric excess) preferably equal to or greater than 90%, more preferably equal to or greater than 95%, preferably equal to or greater than 96%, preferably equal to or greater than 97%, preferably equal to or greater than 98% and most preferably equal to or greater than 99%; and
- a salt content preferably of 20 mole% or less, more preferably of 10 mole% or less, more preferably of 5 mole% or less, more preferably of 2 mole% or less, most preferably of 1 mole% or less, expressed as moles of salt relative to moles of ester.
- a "ratio" as defined hereinbefore of preferably ≥85%, more preferably ≥90%, more preferably ≥95%, more preferably ≥96%, more preferably ≥97%, more preferably ≥98%, most preferably ≥99%.

In step (b) of the process of the invention, cephradine is formed by mixing the mixture obtained in step (a), optionally purified as described hereinbefore with 7-ADCA and a penicillin acylase enzyme, preferably an immobilized enzyme, to form the corresponding semi-synthetic β-lactam compound, all under anaerobic conditions. After the enzymatic coupling, the cephradine can be recovered using known methods. For instance, the enzyme reactor may be discharged through the bottom sieve using upwards stirring. The resulting cephradine suspension may then be filtered through a glass filter. Due to the low amount of DHPG present after the enzymatic coupling reaction, crystallization of the final cephradine may be carried out at high concentrations of the cephradine which results in high yields.

The enzyme that is suitable as a catalyst in reacting 7-ADCA with DHPG in activated form to prepare cephradine in the process according to the invention is known under the general term penicillin acylase, or penicillin G acylase, also called penicillin G amidase or benzylpenicillin acylase (EC 3.5.1.11). Penicillin G acylase refers to a group of hydrolases from microorganisms, especially bacteria, capable of hydrolyzing the 6-acyl group of penicillins or the 7-acyl group of cephalosporins. Penicillin acylase enzymes may be classified both on the basis of their substrate specificity and on the basis of their molecular structure, which is described in various publications, see for instance WO 03/055998 and WO 98/20120.

Microorganisms from which penicillin acylase enzymes may be obtained are for example *Acetobacter,* in particular *Acetobacter pasteurianum, Aeromonas, Alcaligenes,* in particular *Alcaligenes faecalis, Aphanocladium, Bacillus* sp., in particular *Bacillus megaterium, Cephalosporium, Escherichia,* in particular *Escherichia coli, Flavobacterium, Fusarium,* in particular *Fusarium oxysporum* and *Fusarium solani, Kluyvera, Mycoplana, Protaminobacter, Proteus,* in particular *Proteus rettgari, Pseudomonas* and *Xanthomonas,* in particular *Xanthomonas citrii.*

In one embodiment of the invention the enzyme may be immobilized on a carrier. In immobilized form the enzyme can be readily separated and recycled. Immobilized enzymes are known as such and are commercially available, for example an E. coli penicillin acylase isolated as described in WO 92/12782 and immobilized as described in EP222462 and in WO97/04086. The process for preparing cephradine according to the invention may be carried out at any suitable pH and temperature, for instance depending on the enzyme used. For instance, the process comprising reacting 7-ADCA with DHPGa to form cephradine may be carried out in the presence of a wild type penicillin acylase and at a temperature below 15°C. Preferably, the process comprising reacting 7-ADCA with DHPGa to form cephradine in the presence of a wild type penicillin acylase is carried out at a temperature below 15°C and at a pH of at least 7.0.

For enzymes having an increased S/H ratio with respect to the wild-type acylase of E. coli, increased conversions are achieved if the reaction is carried out at relatively high temperature, e.g. above 15°C, preferably between 15 and 30°C and relatively low pH, e.g. below 7.7, preferably between 6 and 7.5. Therefore the present disclosure also relates to a process comprising reacting 7-ADCA with DHPGa under anaerobic conditions to form cephradine which is carried out in the presence of an enzyme being an acylase having a higher S/H ratio preferably a higher S/Hᵢₙᵢ than the wild-type acylase of E.coli and at a temperature of at least 15°C, preferably between 15 and 30°C. Preferably, reacting 7-ADCA with DHPGa to form cephradine in the presence of an enzyme being an acylase having a higher S/H ratio than the wild-type acylase of E. coli is carried out at a temperature of at least 15°C, preferably between 15 and 30°C and at a pH of below 7.7, preferably between 6 and 7.5.

This is surprising as in the case of the enzyme having a relatively low S/H ratio, the highest conversions were found to be achieved at relatively low temperature and relatively high pH. The above effect is in particular pronounced when the acylase having an increased S/H ratio has a lower enzymatic activity than that of the wild-type acylase of E. coli.

The acylase having a higher S/H ratio than the wild-type acylase of E. coli, may be any enzyme. The enzyme may for example be a mutant penicillin acylase. Mutants of penicillin acylases or acylase mutants, can be made by starting from any known penicillin acylase. Mutated acylases are for example derived from wild-type acylases via recombinant DNA methodology known in the art, by substituting one amino acid residue for a new residue.

In a preferred embodiment the enzyme is a mutant penicillin acylase having an amino acid substitution at the position 24 of the β-subunit corresponding to the β-subunit of penicillin acylase of E. coli. In a preferred embodiment, the L-phenylalanine at the position 24 of the β-subunit corresponding to the β-subunit of penicillin acylase of E. coli, has been replaced in that position by L-alanine, as is described in WO 98/20210. This mutation can be applied on a PenG acylase from E. coli, but PenG acylases from other sources may also by used. The numbering of the position of the amino acids corresponds to the numbering of the amino acid sequence of wild type Penicillin G acylase of E. coli.

As defined herein, the synthesis/hydrolysis (S/H) ratio is understood to be the molar ratio of synthesis product to hydrolysis product at a particular moment during the enzymatic reaction. Synthesis product is understood to be the β-lactam antibiotic formed from the activated side chain and β-lactam nucleus. Hydrolysis product is understood to be the corresponding acid of the activated side chain.

The S/H ratio is a function of the concentration of the reactants, the molar ratio of activated side chain to β-lactam nucleus, the temperature, the pH and the enzyme. In the ideal situation a comparative experiment is carried out where the particular candidate is tested against a reference enzyme, preferably E.coli PenG acylase, under the same conditions.

During an enzymatic acylation reaction the S/H ratio generally decreases. The S/H ratio of different penicillin acylases are preferably compared at equal conversion. They are most usually compared at 0% conversion (hence, at time t=0), the so-called initial S/H ratio (S/Hini), which thus is a measure of the S/H ratio. The S/Hini can be determined with sufficient accuracy by carrying out the acylation reaction until a sufficiently high conversion is reached so that the products, in particular the hydrolysis product, can be measured accurately and then constructing a graph of S/Hini versus conversion and extrapolating it to 0% conversion. It might be necessary to determine the initial S/H ratio through extrapolation, since too little hydrolysis product is formed at low conversion for accurate determination of S/Hini. Curve fitting algorithms which are known in the art may be applied to get a more reliable extrapolation. For accurate determination it is necessary to have sufficient data points. Sufficient data points means at least three data points, which should represent a difference in conversion of at least 0.5%.

Enzymatic activity can generally be defined as the molar quantity of reactant or product that is converted or synthesised per unit of time and per quantity of dissolved or immobilised enzyme at a particular moment during the enzymatic acylation reaction. Preferably, enzymes are applied in immobilised form and the enzymatic activity is defined per quantity of immobilised enzyme. The enzymatic activity per quantity of enzyme often is also indicated as specific activity of the particular enzyme.

The process according to the invention may be carried out in several ways, for instance in batch or continuous culture. Preferably, the process according to the invention is carried out in a batch process. A batch culture may be a batch culture, a fed-batch culture, a combination of batch and fed-batch mode, repeated fed-batch mode or any other combination.

The cephradine formed by the process according to the invention may be crystallized in any suitable manner.

Cephradine hydrate having an increased stability is preferably obtained according to the preferred embodiments described hereinafter.

Preferably, the process comprises crystallising the cephradine from an aqueous solution. The aqueous solution may contain an organic solvent or a mixture of organic solvents, preferably less than 30 vol%, more preferably less than 20 vol.%, more preferably less than 10 vol.%, more preferably less than 5 vol.% (relative to the total volume of the liquid). Preferably, the organic solvent is an alcohol with 1-7 carbon atoms, for instance a monoalcohol, in particular methanol or ethanol; a diol, in particular ethylene glycol, or a triol, in particular glycerol. Preferably, the aqueous solution contains at least 70 vol.% water, more preferably at least 80 vol.%, more preferably at least 90 vol.%, most preferably at least 95 vol.% water (relative to the sum volume of the liquid).

The aqueous solution may contain 7-ADCA and/or DHPG.

The cephradine may be crystallised in any suitable form, typically in the form of cephradine hydrate, which is not limited to a specific cephradine hydrate. Typically, the cephradine hydrate is cephradine monohydrate. The water content of the cephradine hydrate may for instance range between 3% and 6% per weight. Preferably, cephradine is crystallised from an aqueous solution wherein the ratio mCEF/(m7-ADCA + mCEF) > 0.7, preferably > 0.8, more preferably > 0.9, and wherein xDH is between 0 and 2 wt.%, preferably between 0 and 1 wt.%, wherein
mCEF = molar quantity of cephradine in the aqueous solution;
m7-ADCA = molar quantity of 7-ADCA in the aqueous solution; and
xDH = concentration of DH in the aqueous solution relative to the total weight of the aqueous solution. The total weight of the aqueous solution includes the weight of the liquid phase and the weight of any solid components present in the aqueous solution, for instance cephradine hydrate.

It was found that the preferred aqueous solution can efficiently be obtained by the process according to the invention resulting in increased conversions and low concentrations DH.

Crystallising cephradine from the preferred aqueous solution was found to result in an improved quality of the crystallised cephradine.

Therefore, the present disclosure also relates to a process comprising reacting 7-aminodesacetoxy cephalosporanic acid (7-ADCA) with D-dihydrophenylglycine in activated form (DHPGa) in the presence of an enzyme in a reaction mixture to form cephradine; and crystallising the cephradine from an aqueous solution, in which aqueous solution the ratio mCEF/(m7-ADCA + mCEF) > 0.7, preferably > 0.8, more preferably > 0.9, and wherein xDH is between 0 and 2 wt.%, preferably between 0 and 1 wt.%.

Preferably, the concentration of 7-ADCA in the aqueous solution is between 0 and 5 wt.%, preferably between 0 and 2 wt.% of the total weight of the aqueous solution. This was found to result in a further improvement of the quality of the crystallised cephradine.

The aqueous solution comprising the cephradine may be prepared in any suitable way.

Preferably, the process for preparing cephradine comprises separating the enzyme from the cephradine prior to said crystallising. The enzyme may for example be separated from the reaction mixture comprising the cephradine, for instance by sieving the reaction mixture over a sieve to separate the enzyme from the cephradine. In the reaction mixture part of the cephradine may be present in the form of cephradine hydrate, and the process may comprise dissolving the cephradine hydrate, and separating the enzyme from the resulting solution. Separating the enzyme from an aqueous solution comprising dissolved cephradine may for example be performed according to any suitable method, such as filtration or centrifugation.

In an embodiment of the process part of the cephradine formed is present in the reaction mixture as cephradine hydrate, and the process further comprises dissolving at least part of said cephradine hydrate.

Cephradine hydrate may be dissolved in any suitable way. Dissolving cephradine hydrate may for example be performed at a pH at or above 8, more preferably, at a pH of between 8.3 and 9.5, and most preferably at a pH of between 8.5 and 9. Or at a pH lower than 3.0, more preferably lower than 2.5, most preferably at pH lower than 2.0 In a preferred embodiment, dissolving cephradine hydrate is for example performed by modifying, in particular by increasing the pH of the reaction mixture to a value at or above 8, preferably, at a pH of between 8.3 and 9.5, and most preferably at a pH of between 8.5 and 9. The pH of the reaction mixture may be increased at the desired pH value by adding a suitable base, for example sodium hydroxide or ammonia. Said dissolving may be carried out batch-wise or continuously. It is also possible to separate cephradine hydrate from the reaction mixture, and to dissolve the separated cephradine hydrate to form the aqueous solution.

Crystallizing cephradine from an aqueous solution comprising cephradine may be carried out at any suitable temperature. Surprisingly we found that improved product quality is achieved when the crystallisation is effected at increased temperature. The cephradine crystallised at these temperatures may have been prepared chemically or enzymatically.

Accordingly, the present disclosure also relates to a process for preparing cephradine crystals, characterised in that the process comprises crystallising cephradine from an aqueous solution to form cephradine crystals, wherein said crystallising is carried out at a temperature of between 45 and 65°C, preferably between 45 and 60°C, preferably between 48 and 55°C. Most preferably, the crystallising is performed at a temperature between 49 and 52°C. Cephradine prepared in an enzymatic process by reacting 7-ADCA with DHPGa in the presence of an enzyme in a reaction mixture to form cephradine, preferably in a process according to the present disclosure may advantageously be crystallised at the temperatures disclosed above. However, the temperatures for crystallisation are not limited to crystallisation of cephradine prepared in an enzymatic process, and may for instance advantageously be applied for crystallisation of cephradine prepared in a chemical process (e.g. in the absence of an enzyme).

Cephradine prepared in an enzymatic process by reacting 7-ADCA with DHPGa in the presence of an enzyme in a reaction mixture to form cephradine, preferably in a process according to the present disclosure, may also be crystallised at a temperature between 35 and 60°C, for instance at a temperature between 35 and 55°C, for instance at a temperature between 35 and 45°C.

Crystallising cephradine from an aqueous solution comprising cephradine may be performed at any suitable pH. Preferably, crystallising the cephradine may be performed at a pH of between 4.0 and 6.0, preferably at a pH of between 4.5 and 5.5, more preferably at a pH of between 4.7 and 5. Surprisingly it was found that at the preferred pH ranges at which crystallisation is performed, the yield of the cephradine crystals was increased. In the framework of the present process, the pH of the aqueous solution may be adjusted in several ways, for instance chemically, by adding an acid, for instance a mineral acid, in particular sulphuric acid, hydrochloric acid or nitric acid. Preferably, said crystallising is performed continuously.

Applying the preferred conditions was surprisingly found to result in cephradine hydrate exhibiting an increased stability in the stability test measured by a decreased absorbance at a wavelength of 450 nm.

The process according to the present disclosure also comprises performing said crystallising at such pH and at such temperature that the absorbance at 450 nm of the cephradine hydrate prepared is below 0.050, preferably below 0.040, and most preferably below 0.030, usually above 0.005.

In one embodiment of the process the enzymatic reaction is carried out in the presence of sodium bisulphite. Preferably, the amount of sodium bisulphite present in the enzymatic reaction is between 1 and 25 mM, preferably between 5 and 15 mM. Surprisingly, the presence of sodium bisulphite during the enzymatic reaction further decreased the coloration of the cephradine prepared.

Sodium bisulphite may also be present during crystallisation of cephradine in the process according to the invention. Preferably, the amount of sodium bisulphite present during crystallising of cephradine to form cephradine monohydrate crystals is between 5 and 250 mM, more preferably between 25 and 150 mM. The cephradine hydrate may be separated from the aqueous solution and dried in any suitable manner.

The cephradine hydrate obtainable by the process as described herein preferably has the following properties:
a. ≤5% cephalexin, more preferably ≤4% cephalexin, more preferably ≤5% cephalexin even more preferably ≤2% cephalexin and most preferably ≤1% cephalexin, and/or
b. an absorbance at 450 nm of below 0.2, preferably below 0.15, more preferably below 0.1, more preferably below 0.05. Preferably the cephradine hydrate has an absorbance of between 0.001 and 0.2, preferably between 0.002 and 0.15, more preferably between 0.004 and 0.1, more preferably between 0.005 and 0.05, and more preferably of between 0.010 and 0.040; and/or
c. a high color stability in the stress stability test, i.e. the coloration of the cephradine hydrate is below 0.20, more preferably below 0.15, and most preferably below 0.10 at an absorbance of 450 nm after 8 weeks of storage at 40°C at a relative humidity of 75%; and/or
d. contains no, or substantially no organic solvents. Preferably the cephradine hydrate contains ≤100 ppm DMF, more preferably ≤75 ppm DMF, more preferably ≤50 ppm DMF, more preferably ≤25 ppm DMF, even more preferably ≤10 ppm DMF and most preferably no detectable DMF and/or the cephradine hydrate contains ≤200 ppm DCME, more preferably ≤150 ppm DCME, more preferably ≤100 ppm DCME, more preferably ≤50 ppm DCME, more preferably ≤25 ppm DCME, even more preferably ≤10 ppm DCME and most preferably no detectable DCME

The cephradine hydrate according to the invention may have any combination of properties (a) to (d) listed above for instance a+b or a+c or a+d or b+c or b+d or c+d, a+b+c or a+b+d or a+c+d or b+c+d or a+b+c+d. Preferred are composition with at least property a: a+b or a+c or a+d or a+b+c or a+b+d or a+c+d or most preferred a+b+c+d.

### MATERIALS AND METHODS

Nitrogen gas from the grid was used without further purification. DHPG was obtained from Shanghai Comfort Biochemical Co.

### EXAMPLES

### Comparative example

### a) Synthesis of D-dihydrophenylglycine-methylester (DHPGM) solution

153 g DHPG with a content of 99.11% as measured by potentiometric assay and with a D-phenylglycine (PG) content of 0.58%, was suspended in 280 ml methanol and 121 g concentrated sulfuric acid was dosed in 60 minutes (during the dosing the temperature was kept at 15-20°C). The mixture was kept at reflux for 2 hours and concentrated at reduced pressure (p=100 mBar, T = 75-80°C). Then, 140 ml methanol was added and the mixture was kept again at reflux for 1 hour and concentrated at reduced pressure. The procedure was repeated another six times. Finally, 140 ml methanol was added; the solution was refluxed for another hour and cooled to ambient temperature. The pH was increased to 2.1 with concentrated ammonia in 15 minutes at 20°C. 87 ml water was added. Methanol was distilled at reduced pressure and 40-50°C. Finally 374 g solution was obtained with a pH of 2.0 and containing 40.87 % DHPGM and 2.0% PGM.

### b) Enzymatic synthesis of Cephradine

A reactor with a 175 µm sieve bottom was filled with 37 g immobilized *Escherichia coli* PenG acylase mutant Phe-B24-Ala. Subsequently 22.3 g 7-ADCA, 0.03 g EDTA and 50 g water were added at 20°C and the pH was adjusted to 7.2 with 25% ammonia. 46 g DHPGM solution as obtained in step a) (above) was dosed into the reactor at a constant rate in 180 min. The pH was maintained at 7.2 with ammonia. The temperature was kept at 20°C. After 60 min, 0.1 g solid cephradine (seed) was added. After 240 min, the pH was decreased to 6.0 with 25% sulfuric acid.

### c) Recovery of cephradine

The reactor was discharged through the bottom sieve with upwards stirring. The resulting cephradine suspension was filtered through a glass filter. The resulting mother liquor was transferred back into the reactor. This sequence of steps was repeated five times. Subsequently, the enzyme was washed with 3 x 10 ml water. The cephradine wet cake, mother liquors and wash waters were combined, and the temperature was decreased to 2°C. The pH was decreased to 1.8 with 25% sulfuric acid and the resulting solution was filtered through a 0.45 µm filter. The filtered cephradine solution was heated to 48°C, the pH increased to 2.6 with concentrated ammonia and 1.0 g solid cephradine (seed) was added. After 15 minutes the pH was increased to 4.8 in 40 minutes. Subsequently, the suspension was stirred at 20°C for another 30 min. The suspension was filtered through a glass filter and the wet cake was washed with 2 x 20 ml water and 2 x 25 ml acetone. After drying, 19.0 g cephradine monohydrate was obtained with a cephalexin content of 7.3 %.

### Example 1

### a) General; preparation of raw materials

Nitrogen gas was purified by filtration through a Varian CP17971 moisture filter and a Varian CP17970 filter, successively. Sulfuric acid, methanol, water and enzymes were degassed with nitrogen before use, until the oxygen content in the outlet had become below 100 ppm. DHPG was used with a PG content of 1.14%, after 13 months storage. The complete synthesis was done in closed equipment and under nitrogen.

### b) Synthesis of DHPGM solution

153 g DHPG with a content of 99.11% as measured by potentiometric assay and with a D-phenylglycine (PG) content of 1.14%, was suspended in 280 ml methanol, and the suspension was degassed with nitrogen. 121 g concentrated sulfuric acid was dosed in 60 minutes (during the dosing the temperature was kept at 15-20°C). The mixture was kept at reflux for 2 hours and concentrated at reduced pressure (p = 100 mBar, T = 75-80°C). Breaking of vacuum (pressure increase from vacuum to atmospheric) was done with nitrogen. 140 ml methanol was added and the mixture was kept again at reflux for 1 hour and concentrated at reduced pressure. The procedure was repeated for another six times. Finally, 140 ml methanol was added; the solution was refluxed for another hour and cooled to ambient temperature.

The pH was increased to 2.1 with concentrated ammonia in 15 minutes at 20°C. 87 ml water was added. Methanol was distilled at reduced pressure and 40-50°C. Finally 374 g solution was obtained with a pH of 2.0, and containing 44.96 % DHPGM and 0.87% PGM. Apparently, when the synthesis is carried out under nitrogen, less PGM (0.87%) is formed compared to the comparative example (2.00%).

### c) Enzymatic synthesis of Cephradine

A reactor with a 175 µm sieve bottom was filled with 37 g immobilized *Escherichia coli* PenG acylase mutant Phe-B24-Ala. 22.3 g 7-ADCA, 0.03 g EDTA, 0.4 g sodium persulfate and 50 g water were added at 20°C and the suspension was degassed with nitrogen. The pH was adjusted to 7.2 with 25 % ammonia.

46 g DHPGM solution as obtained in step b) (above) was dosed into the reactor at a constant rate in 180 min. The pH was maintained at 7.2 with ammonia. The temperature was kept at 20°C. After 60 min, 0.1 g solid cephradine (seed) was added. After 240 min, the pH was decreased to 6.0 with 80% sulfuric acid.

### d) Recovery of cephradine

The reactor was discharged through the bottom sieve with upwards stirring. The resulting cephradine suspension was filtered through a glass filter. The resulting mother liquor was transferred back into the reactor. This sequence of steps was repeated five times. Subsequently, the enzyme was washed with 3 x 10 ml water. The cephradine wet cake, mother liquors and wash waters were combined, and the temperature was decreased to 2°C. The pH was decreased to 1.8 with 80% sulfuric acid and the resulting solution was filtered through a 0.45 µm filter. The filtered cephradine solution was heated to 48°C, the pH increased to 2.6 with concentrated ammonia and 1.0 g solid cephradine (seed) was added. After 15 minutes the pH was increased to 4.8 in 40 minutes. Subsequently, the suspension was stirred at 20°C for another 30 min. The suspension was filtered through a glass filter and the wet cake was washed with 2 x 20 ml water and 2 x 25 ml acetone. After drying, 25.6 g cephradine monohydrate was obtained with a cephalexin content of 1.74%.

### Example 2

This experiment was done in the same way as example 1, but starting with freshly prepared DHPG with a PG content of 0.63%. In the end 25.6 g cephradine monohydrate was obtained with a cephalexin content of 1.03 %.

## Claims

1. Process for preparing cephradine, said process comprising
a. converting D-dihydrophenylglycine (DHPG) into an activated form (DHPGa) which is an amide or an ester; and
b. reacting 7-aminodesacetoxy-cephalosporanic acid (7-ADCA) with D-dihydrophenylglycine in activated form (DHPGa) in the presence of a penicillin acylase in an aqueous reaction mixture to form cephradine,
**characterized in that** at least step (a) and step (b) of the process are carried out under anaerobic conditions.

2. Process according to claim 1 wherein the anaerobic conditions are created by
a. carrying out step (a) and step (b) of the process in the presence of one or more oxygen scavengers;
b. degassing one or more of the raw materials, reagents, solvents and/or water used in step (a) and step (b) of the process of claim 1;
c. carrying out step (a) and step (b) of the process of claim 1 in an atmosphere comprising an inert gas; or
d. a combination of a+b, a+c, b+c or a+b+c.

3. Process according to claim 2 wherein the degassing is obtained by flushing the one or more of the raw materials, reagents, solvents and water with nitrogen gas.

4. Process according to claim 3 wherein step (a) comprises converting DHPG with an alcohol to form a mixture comprising the corresponding DHPG ester.

5. Process according to claim 4 wherein the alcohol is methanol or ethanol, preferably methanol.

6. Process according to any of the preceding claims wherein said reacting in step (b) results in a conversion of 7-ADCA into cephradine of at least 70% and wherein the concentration of DHPG in the reaction mixture is below 2 wt.%.

7. Process according to claim 4 **characterized in that** the DHPG-ester formed in step (a) is not isolated as a solid intermediate prior to carrying out step (b).

## Patentansprüche

1. Verfahren zur Herstellung von Cephradin, wobei das Verfahren Folgendes umfasst:
a. Umwandeln von D-Dihydrophenylglycin (DHPG) in eine aktivierte Form (DHPGa), bei der es sich um ein Amid oder einen Ester handelt; und
b. Umsetzen von 7-Aminodesacetoxycephalosporansäure (7-ADCA) mit D-Dihydrophenylglycin in aktivierter Form (DHPGa) in Gegenwart einer Penicilinacylase in einer wässrigen Reaktionsmischung, wodurch Cephradin gebildet wird,
**dadurch gekennzeichnet, dass** mindestens Schritt (a) und Schritt (b) des Verfahrens unter anaeroben Bedingungen durchgeführt werden.

2. Verfahren nach Anspruch 1, wobei die anaeroben Bedingungen durch Folgendes erzeugt werden:
a. Durchführen von Schritt (a) und Schritt (b) des Verfahrens in Gegenwart von einem oder mehreren Sauerstofffängern;
b. Entgasen von einem oder mehreren der in Schritt (a) und Schritt (b) des Verfahrens von Anspruch 1 verwendeten Rohmaterialien, Reagenzien, Lösungsmittel und/oder Wasser;
c. Durchführen von Schritt (a) und Schritt (b) des Verfahrens von Anspruch 1 in einer Atmosphäre, die ein Inertgas umfasst; oder
d. eine Kombination von a+b, a+c, b+c oder a+b+c.

3. Verfahren nach Anspruch 2, wobei das Entgasen durch Spülen von dem einen oder den mehreren der Rohmaterialien, Reagenzien, Lösungsmittel und Wasser mit Stickstoffgas erzielt wird.

4. Verfahren nach Anspruch 3, wobei Schritt (a) das Umwandeln von DHPG mit einem Alkohol umfasst, wodurch eine Mischung, die den entsprechenden DHPG-Ester umfasst, gebildet wird.

5. Verfahren nach Anspruch 4, wobei es sich bei dem Alkohol um Methanol oder Ethanol, vorzugsweise Methanol, handelt.

6. Verfahren nach einem der vorhergehenden Ansprüche, wobei das Umsetzen in Schritt (b) zu einer mindestens 70%igen Umsetzung von 7-ADCA zu Cephradin führt und wobei die DHPG-Konzentration in der Reaktionsmischung unter 2 Gew.-% liegt.

7. Verfahren nach Anspruch 4, **dadurch gekennzeichnet, dass** der in Schritt (a) gebildete DHPG-Ester vor der Durchführung von Schritt (b) nicht als festes Zwischenprodukt isoliert wird.

## Revendications

1. Procédé pour la préparation de céphradine, ledit procédé comprenant
a. convertir de la D-dihydrophénylglycine (DHPG) en une forme activée (DHPGa) qui est un amide ou un ester ; et
b. faire réagir de l'acide 7-aminodésacétoxy-céphalosporanique (7-ADCA) avec la D-dihydrophénylglycine sous forme activée (DHPGa) en présence de pénicilline acylase dans un milieu de réaction aqueux afin de former de la céphradine, **caractérisé en ce qu'**au moins les étapes (a) et (b) du procédé sont réalisées dans des conditions anaérobies.

2. Procédé selon la revendication 1, dans lequel les conditions anaérobies sont créées par
a. la réalisation de l'étape (a) et l'étape (b) du procédé en présence d'un ou plusieurs désoxygénants ;
b. le dégazage d'un ou plusieurs éléments parmi les matières premières, les réactifs, les solvants et/ou l'eau utilisés dans l'étape (a) et l'étape (b) du procédé de la revendication 1 ;
c. la réalisation de l'étape (a) et l'étape (b) du procédé de la revendication 1 dans une atmosphère comprenant un gaz inerte ; ou
d. une combinaison de a+b, a+c, b+c ou a+b+c.

3. Procédé selon la revendication 2, dans lequel le dégazage est obtenu en soumettant le ou les éléments parmi les matières premières, les réactifs, les solvants et/ou l'eau à un barbotage à l'azote.

4. Procédé selon la revendication 3, dans lequel l'étape (a) comprend la conversion du DHPG avec un alcool afin de former un mélange comprenant l'ester de DHPG correspondant.

5. Procédé selon la revendication 4, dans lequel l'alcool est le méthanol ou l'éthanol, de préférence le méthanol.

6. Procédé selon l'une quelconque des revendications précédentes, dans lequel ladite réaction à l'étape (b) donne lieu à une conversion de 7-ADCA en céphradine d'au moins 70 % et dans lequel la concentration de DHPG dans le mélange réactionnel est inférieure à 2 % en poids.

7. Procédé selon la revendication 4, **caractérisé en ce que** l'ester de DHPG formé à l'étape (a) n'est pas isolé sous la forme d'un produit intermédiaire solide avant la réalisation de l'étape (b).
